# EUROPEAN PATENT APPLICATION

(11) **EP 4 813 380 A1**
(43) Date of publication of application: **30.09.2026**
(21) Application number: 26166851.1
(22) Date of filing: 23.03.2026
(51) Int. Cl.: A61K 31/16, A61K 31/497, A61K 31/56, A61K 31/661, A61P 35/00, A61P 35/02, A61P 35/04

(54) **EDELFOSINE FOR USE IN THE TREATMENT OF MULTIPLE MYELOMA**

(30) Priority: 25.03.2025 US 202563777263 P
(71) Applicant: RDP Pharma AG, 8590 Romanshorn (CH)
(72) Inventor: ZANDER, Axel, Potsdam (DE); MEYER, Wolfgang, Munich (DE); ATANACKOVIC, Djordje, Baltimore (US)
(74) Representative: Forstmeyer, Dietmar

(57) **Abstract**

The present invention relates to the use of the alkyl phospholipid edelfosine (especially (S)-edelfosine) in the treatment of multiple myeloma.

## Description

The present invention relates to the use of the alkyl phospholipid edelfosine (CP201; especially (S)-edelfosine, S-CP201) in the treatment of multiple myeloma.

Multiple myeloma (MM) is a clonal plasma cell proliferative disorder belonging to the monoclonal gammopathies characterized by the abnormal increase of monoclonal immunoglobulins (paraprotein) leading to evidence of specific end-organ damage. MM is a very heterogeneous disease as per biological, clinical, and prognostic points of view with a median survival ranging from two to more than 10 years; it is considered an incurable disease characterized by remission periods alternated with relapse/progression episodes, finally leading to resistant disease. The consequences of undiagnosed and untreated disease are severe. The excess of monoclonal immunoglobulins results in blood hyperviscosity, platelet dysfunction, and renal tubular damage, leading to neurologic derangements, bleeding, and renal failure, respectively. Anemia, thrombocytopenia, leukopenia, and bone loss are also common (Albagoush et al, 2023; Cowan et al, 2022; Rajkumar, 2024).

The exact etiology of MM is unknown. However, frequent alterations and translocations in the promoter genes, especially chromosome 14, are commonly found in MM. In addition, other oncogenes such as NRAS, KRAS, and BRAF may participate in plasma cell proliferation. Other factors contributing to disease occurrence include obesity, alcohol consumption, and environmental factors (e.g., certain insecticides, organic solvents, and radiation exposure) (Albagoush et al, 2023).

MM is relatively uncommon and only represents about 1.8% of all new cancer cases diagnosed in the United States each year. It occurs predominantly in the geriatric population with a median age at diagnosis of about 70 years and is slightly more commonly seen in males than females (1.4:1) (Cowan et al, 2022; Rajkumar, 2024).

Diagnostic measures include bone marrow aspirate and biopsy, immunohistochemistry, extensive blood chemistry evaluations, plasma cell FISH, skeletal radiography, CT/ PET-CT, and MRI (Albagoush et al, 2023; Cowan et al, 2022; Rajkumar, 2024).

The prognosis for multiple myeloma is quite variable, and many factors can affect outcomes. Two main drivers in prognosis are stage and disease biology. MM staging systems (e.g., R-ISS staging) have been developed to better predict outcomes. Per R-ISS, Stage III demonstrated a five-year overall survival of 40% and progression-free survival of 24%. Also, certain cytogenetic abnormalities (e.g., 4:14), t(14:16), and f(14:20)) are associated with a poor prognosis (Albagoush et al, 2023).

Treatment for multiple myeloma is focused on decreasing the clonal plasma cell population and consequently decrease the symptoms of disease.

Initial management of MM should involve evaluation for any acute issues that must be stabilized immediately (e.g., administration of saline for volume expansion, treatment of severe hypercalcemia or renal dysfunction). Once stabilized, the patient's treatment plan is driven by risk stratification and transplant eligibility. A patient's disease can fall into one of two categories: high or standard-risk MM (defined by genetic markers) (Albagoush et al, 2023; Cowan et al, 2022; Rajkumar, 2024).

Standard-risk patients who are fit for transplantation often receive a cytotoxic induction therapy (e.g., daratumumab, bortezomib, lenalidomide, and dexamethasone; see below) over 3 or 4 months to decrease the tumor burden, followed by an autologous stem cell transplant (ASCT). Thereafter, the transplanted patient will be placed back on to a maintenance therapy (e.g., lenalidomide) until disease progression or based on tolerability.

For high-risk (mostly elderly), transplant-ineligible patients, there are several options. One approach would be to employ the VRd regimen (bortezomib, lenalidomide, and dexamethasone for 8 to 12 cycles followed by maintenance bortezomib-based therapy). Another option could include daratumumab, lenalidomide, and dexamethasone (DRd) continued until disease progression (Albagoush et al, 2023; Cowan et al, 2022; Rajkumar, 2024).

Next to ASCT, medical treatment is the basis of treating MM. Various compounds have been licensed to treat MM, addressing different disease settings. As indicated, most drug therapies employ multiple agents, e.g., so-called "triplet" or "quadruplet" therapies. Many such groupings include one or more of a monoclonal antibody (e.g., isatuximab or daratumumab), an immunomodulatory agent (e.g., lenalidomide or pomalidomide), and a proteasome inhibitor (e.g., or bortezomib, carfilzomib or ixazomib), in combination with a corticosteroid (e.g., dexamethasone). While triplet therapies (e.g., VRd, PDd [bortezomib, liposomal doxorubicin and dexamethasone]) were the standard of care for many years, current practice more commonly applies quadruplets of drugs (Albagoush et al, 2023).

Commonly, the efficacy of each drug diminishes over time, as MM develops drug resistance mechanisms, such as by clonal evolution or genetic mutations. Thus, patients may undergo "watchful waiting" while the disease is smoldering or is asymptomatic, to preserve the drug(s) efficacy for the time when the disease progresses. Also, maintenance therapy using a prolonged course of low-toxicity medications (e.g., lenalidomide or bortezomib) are given to reduce the risk of relapse (Albagoush et al, 2023).

Eventually, most people, including those treated with ASCT, relapse after initial treatment. Relapse within the first 18 months of diagnosis is considered as functional high-risk multiple myeloma. Depending on the person's condition, the prior treatment modalities used, and the duration of remission, options for relapsed disease include retreatment with the original agent, use of other agents (such as melphalan, cyclophosphamide, thalidomide, or dexamethasone, alone or in combination), and a second ASCT.

Later in the course of the disease, MM becomes refractory (resistant) to formerly effective treatment. This stage is referred to as relapsed/refractory multiple myeloma (RRMM). Treatment modalities that are commonly used to treat RRMM include dexamethasone, proteasome inhibitors (e.g. bortezomib and carfilzomib), immunomodulatory imide drugs (e.g. thalidomide, lenalidomide, and pomalidomide), and certain monoclonal antibodies (e.g. isatuximab against CD38 or antibodies targeting CD319). Survival expectancy has risen in recent years, and new treatments are under development (Cowan et al, 2022).

The introduction of immunomodulators ("imids") (e.g., lenalidomide), proteasome inhibitors (e.g., bortezomib), and biphosphonates (e.g., zoledronic acid) next to traditionally used chemotherapeutic drugs and corticosteroids in the 2000s significantly improved the outcomes for MM. During the recent years, numerous other drugs have been licensed to treat MM, including monoclonal antibodies targeting SLAMF7 (CD319) (e.g., elotuzumab) or CD38 (e.g., daratumumab), histone deacetylase inhibitors (e.g., panobistat), stem cell mobilizing drugs (e.g., plerixafor), and nuclear export inhibitors/exportin 1 (XPO1) inhibitors (e.g., selinexor) (Cowan et al, 2022).

Increasingly, precision medicine therapies are being explored, with research indicating that certain variants and genetic sub-types of the disease respond more favorably to some drug therapies than others. For instance, more recently, BCMA-directed CAR-T cell treatments (e.g., ciltacabtagene autoleucel and idecabtagene vicleucel) have been introduced to treat MM. Also, T cell engaging bispecific monoclonal antibodies addressing BMCA/CD3 (e.g., elranatamab, teclistamab, linvoseltamab) or GPRC5D/CD3 (e.g., talquetamab) have been licensed to treat later stages of MM (Albagoush et al, 2023; Rajkumar, 2024).

Drugs under development to address MM encompass Bcl-2 inhibitors (e.g., venetoclax), PD-1/PD-L1 inhibitors (e.g., pembrolizumab), PI3K inhibitors (e.g., pictilisib), BRAF inhibitors, RAS inhibitors, MEK inhibitors, and MYC-degrading and inhibiting drugs (Offidani et al, 2021).

Edelfosine showed anti-neoplastic activity in six MM cell lines (RPMI 8226/S, 8226/DOX40, 8226/LR-S, 8226/MR-20, 8226/Dox1V, and 8226/MDR10V). On average, IC₅₀ values were 6 µg/mL (Glasser et al, 1996).

In another report, edelfosine triggered strong apoptosis-inducing effects in the following MM cell lines exposed to 10 µM of the drug: MM144, MM1S, MM1R, RPMI-8226, OPM2, and OPM-2 (FAS-deficient) as well as freshly prepared cells from MM patients (Gajate and Mollinedo, 2007; Mollinedo et al, 2010).

Oral daily administration of edelfosine over 30 days (30 mg/kg) resulted in a significant reduction of human MM1S tumor cells transplanted into CB17-SCID mice. Tumor weight and volume were decreased by approximately 88% (Mollinedo et al, 2010).

During the last decades, considerable progress has been made to MM. Numerous new compounds with different mechanisms of action have been approved and introduced into clinical practice.

MM is frequently associated with the elderly, as the majority of the patients are diagnosed between the age of 60 and 70; however, in recent years younger patients have been also diagnosed. The median overall survival increased from 2-3 years to 8-10 years (Pinto et al, 2020). Following the American Cancer Society (SEER Database), the overall 5-year survival rate for MM is 62%, for Stage III MM it is less than 50%. The disease is considered as not curable, and most patients die within 10 years. MM treatment is mostly combination treatment (e.g., quadruple combination composed of daratumumab, bortezomib, lenalidomide, and dexamethasone).

One of the major, still unresolved problem of MM is that patients develop resistance to standard drugs used to treat this disease (e.g., against dexamethasone, bortezomib, or lenalidomide) (Uckun 2022). This is of particular importance in the context of MM maintenance treatment (Holstein et al, 2021). Various mechanisms have been proposed to explain drug resistance in this patient setting, including increased activity of drug transporters, extended degradation of target proteins, mutations of MM driver genes, changed tumor microenvironment, epigenetic alterations, and extended persistence of cancer stem cells (Pinto et al, 2020).

It has been the object of the present invention to provide new treatments for multiple myeloma.

It was shown that S-CP201 exhibits anti proliferative activity against various MM cell lines in vitro.

The following unexpected observation of the present invention is of special importance: it was found that S-CP201 in vitro could revert the activity of dexamethasone in MM cells deemed refractory to dexamethasone.

Dexamethasone has been a mainstay of treatment for MM for decades. In current international treatment protocols and clinical practice guidelines, dexamethasone is included in all phases of MM treatment as a key adjunct to novel therapies within all preferred therapy regimen, augmenting clinical response rates to these agents (Rosenberg, 2023).

Thus, prior treatment with S-CP201 is able to resensitize dexamethasone-refractory MM to dexamethasone again, and can thus open the door for new treatment modalities, for instance in the context of MM maintenance therapy.

The present invention provides edelfosine (especially (S)-edelfosine, S-CP201) for use in the treatment of multiple myeloma.

The present invention further provides edelfosine (especially (S)-edelfosine, S-CP201) for use in the treatment of frontline and advanced or recurrent stages of multiple myeloma.

The present invention moreover provides edelfosine (especially (S)-edelfosine, S-CP201) for use in the treatment of multiple myeloma in standard-risk and high-risk multiple myeloma patients.

The present invention further provides edelfosine (especially (S)-edelfosine, S-CP201) for use as induction treatment for autologous stem cell transplantation (ASCT).

The present invention moreover provides edelfosine (especially (S)-edelfosine, S-CP201) for use as maintenance therapy for multiple myeloma.

The present invention further provides edelfosine (especially (S)-edelfosine, S-CP201) for use in resensitizing dexamethasone-refractory multiple myeloma.

As stated above, the present invention provides edelfosine for use in the treatment of multiple myeloma.

Preferably, the edelfosine for use in the present invention is (S)-edelfosine (or S-CP201)

Moreover preferably, the multiple myeloma that is treated according to the present invention is selected from frontline and advanced or recurrent stages of gliomas.

Further preferably, edelfosine is used in combination with corticosteroids (e.g., dexamethasone, prednisone), proteasome inhibitors (e.g., bortezomib, carfilzomib, ixazomib), cytotoxic drugs (e.g., cyclophosphamide, doxorubicin, bendamustine, melphalan), immunomodulators (e.g., lenalidomide, pomalidomide, thalidomide), stem cell mobilizing drugs (e.g., motixafortide, plerixafor), nuclear export inhibitors/exportin 1 (XPO1) inhibitors (e.g., Selinexor), Bcl-2 inhibitors (e.g., venetoclax), bisphosphonates (e.g., zoledronic acid), SLAMF7 (CD319)-directing drugs (e.g., elotuzumab), histone deacetylase inhibitors (e.g., panobistat), C38-directing drugs (e.g., belantamab, daratumumab, isatuximab), BCMA-directed CAR-T cell treatments (e.g., ciltacabtagene autoleucel, idecabtagene vicleucel), BMCA/CD3 directed bispecific antibodies/T cell engagers (e.g., elranatamab, teclistamab, linvoseltamab), GPRC5D/CD3-directed bispecific antibodies/T cell engagers (e.g., talquetamab), PD-1/PD-L1 inhibitors (e.g., pembrolizumab), Pi3K inhibitors (e.g., pictilisib), BRAF inhibitors, RAS inhibitors, MEK inhibitors, and/or MYC-degrading and inhibiting drugs.

It is further preferred to combine the above embodiments of the present invention in any desired manner.

The present invention further provides pharmaceutical compositions comprising edelfosine or a pharmaceutically acceptable salt thereof, optionally in combination with one or more carrier substances and/or one or more adjuvants.

In general, the compounds and pharmaceutical compositions described herein will be administered by using the known and acceptable modes known in the art.

The compounds and pharmaceutical compositions of the present invention can be administered by one of the following routes:
- oral, e.g. as tablets (including extended-release), mini tablets, sublingual tablets, buccal tablets, dragees, coated tablets, pills, semisolids, capsules (including extended-release), soft or hard capsules, for example soft and hard gelatine capsules, aqueous or oily solutions, emulsions, suspensions, or syrups, juices or drops (e.g., for children);
- parenteral including intravenous, intramuscular and subcutaneous injection, e.g. as an injectable solution or suspension, pre-filled syringes, or infusion solution concentrates (vials);
- rectal as suppositories;
- by inhalation;
- transdermal or topical, for example via a transdermal delivery system (TDS) such as a plaster containing the active ingredient, or as ointments, creams, or lotions.

For the production of such tablets, mini tablets, sublingual tablets, buccal tablets, pills, semisolids, coated tablets, dragees and hard, e.g. gelatine, capsules the therapeutically useful product may be mixed with pharmaceutically inert, inorganic or organic excipients as are e.g. lactose, sucrose, glucose, gelatine, malt, silica gel, starch or derivatives thereof, talc, stearinic acid or their salts, dried skim milk, magnesium-alumininum silicates (e.g. Neusilin), 6-O-a-isomaltulose (Isomalt), cross linked polyvinyl N-pyrrolidone (PVP, Crosspovidone), magnesium stearate and the like. For the production of soft capsules, one may use excipients as are e.g. vegetable, petroleum, animal or synthetic oils, wax, fat, and polyols. For the production of liquid solutions, emulsions or suspensions or syrups one may use as excipients e.g. water, alcohols, aqueous saline, aqueous dextrose, polyols, glycerin, lipids, phospholipids, cyclodextrins, vegetable, petroleum, animal or synthetic oils. Especially preferred are lipids and more preferred are phospholipids (preferred of natural origin; especially preferred with a particle size between 300 to 350 nm) preferred in phosphate buffered saline (pH = 7 to 8, preferred 7.4). For suppositories one may use excipients as are e.g. vegetable, petroleum, animal or synthetic oils, wax, fat and polyols. The pharmaceutically useful agents may also contain additives for conservation, stabilization, e.g. UV stabilizers, emulsifiers, sweetener, aromatizers, salts to change the osmotic pressure, buffers, coating additives and antioxidants. If the pharmaceutical compositions are intended to be administered via the oral route to children, it is preferred that they are formulated as a syrup, a sublingual tablet or a fast-dissolving tablet. Preferably, formulations for oral administration to children have a pleasant taste.

### Examples

### 1. In vitro studies conducted with S-CP201 in MM cell lines

In an in vitro screening experiment including 14 different cell lines from various tumor types, GI₅₀ values of at or below 1 µM were measured for MM cell lines KMS-34, NCI-H929, OPM-2 and RPMI-8226, and < 2 µM for MM cell line EJM.

Two of these cell lines (KMS-34 and RPMI-8226) already display onset of cytotoxicity between 72h and 120h, indicating high sensitivity for S-CP201.

A combination of S-CP201 with either the proteasome inhibitor carfilzomib or the immunomodulator pomalidomide shows distinct synergy in select cell lines, with the multiple myeloma cell line OPM-2 displaying the highest capacity for synergy.

### 2. In vitro single-agent and combination studies with S-CP201 in four MM cell lines

Several in vitro experiments were conducted to investigate the anti-proliferative effects of S-CP201 in several multiple myeloma (MM) cell lines, either alone or in combination with the approved medications dexamethasone (immunosuppressive, IL-6 inhibitor), selixenor (exportin 1 [XPO1] inhibitor), and ixazomib (proteasom inhibitor). The single-agent activity of the latter three compounds was also tested. Different concentrations of S-CP201 and the combination partners were assessed. The following cell lines were used: RPMI-8226, MM1S, and ANBL-6.

Cell line RPMI-8226 was chosen based on prior experiences. Cell lines MM1S, MM1R and ANBL-6 were added since they were shown to have more similarities with the genome and transcriptome profiles of MM primary patient tumors as well as regarding their response to treatment with MM drugs.

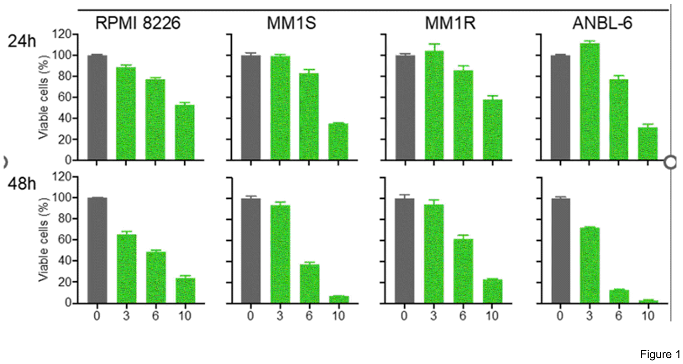

The concentrations chosen for the S-CP201 monotherapy treatment (3, 6 and 10 µM) covered a range from a reported therapeutic, non-cytotoxic (3 µM) to a cytotoxic concentration (10 µM) (see Figure 1). S-CP201 exhibited marked dose-dependent single-agent anti-proliferative activity in the four tested cell lines. Single agent activity of S-CP201 could be demonstrated starting at 6 µM in all 4 cell lines (Figure A), starting at 24 hours and being markedly more pronounced at 48 hours (40% reduction of viable cells for cell lines RPMI-8226 and MM1R, and 60 and 80% for MM1S and ANBL-6, respectively). It should be noted that ANBL-6 is a difficult-to-grow, interleukin-6 (IL-6) dependent cell line which is close to the clinical setting. IL-6 is considered an important growth and survival factor for MM.

Figure 1 shows in vitro efficacy of S-CP201 alone in 4 MM cell lines at 24 and 48 hours at 4 different concentrations (x-axis: concentration of S-CP201 (µM)).

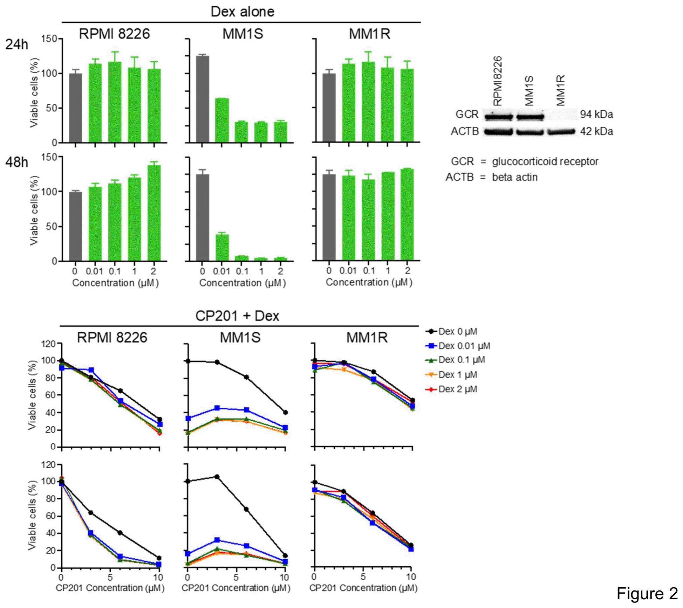

When combined with dexamethasone, selinexor or ixazombib, S-CP201 exhibited a potentiation of the therapeutic effect only in MM cell line RPMI-8226, but not in the cell lines MM1S and MM1R (dexamethasone) or MM1S (selinexor). The combination S-CP201/ixazombib was only tested in RPMI-8226 cells (see Figures 2, 3, and 4).

These results strengthen the fact that different MM cell lines respond differently to the standards of care.

When the different myeloma cell lines were treated with the established anti-myeloma drug dexamethasone in combination with S-CP201, it was observed that the simultaneous treatment with both drugs led to a synergistic anti-myeloma effect at both 24h and 48h for the steroid-sensitive cell line MM1S. In the case of the steroid receptor-loss variant MM1R, the addition of dexamethasone did not lead to a further enhancement of the anti-myeloma efficacy of S-CP201. However, in the case of steroid-resistant myeloma cell line RPMI8226, treatment of S-CP201 for 48 hours led to a resensitization of the cell line to dexamethasone and, as a result, to a synergistic effect of the combined treatment. When the different myeloma cell lines were exposed to either selinexor or ixazomib, two established oral anti-myeloma drugs, in combination with S-CP201, it was found that at least additive and dose-dependent effect of the two-drug combinations on all myeloma lines tested at 24 hours and 48 hours, when compared with the respective single-agent S-CP201 treatment.

Figure 2 shows in vitro efficacy of dexamethasone alone and in combination with S-CP201 in 3 MM cell lines at 24 and 48 hours (Dex=dexamethasone; CP201 = S-CP201; Tested concentrations of dexamethasone alone: 0. 0.01, 0.1. 1 and 2 µM; Tested concentrations of S-CP201 in combination with dexamethasone: 0, 3, 6 and 10 µM).

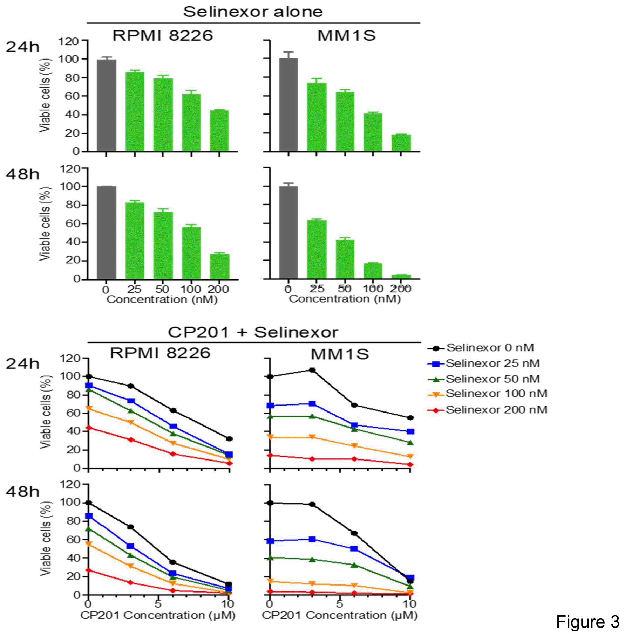

Figure 3 shows in vitro efficacy of selinenor alone and in combination with S-CP201 in 2 MM cell lines at 24 and 48 hours, both tested in different concentrations (CP201 = S-CP201; Tested concentrations of selinexor alone: 0, 25, 50, 100 and 200 nM; Tested concentrations of S-CP201 in combination with selinexor: 0, 3, 6 and 10 µM).

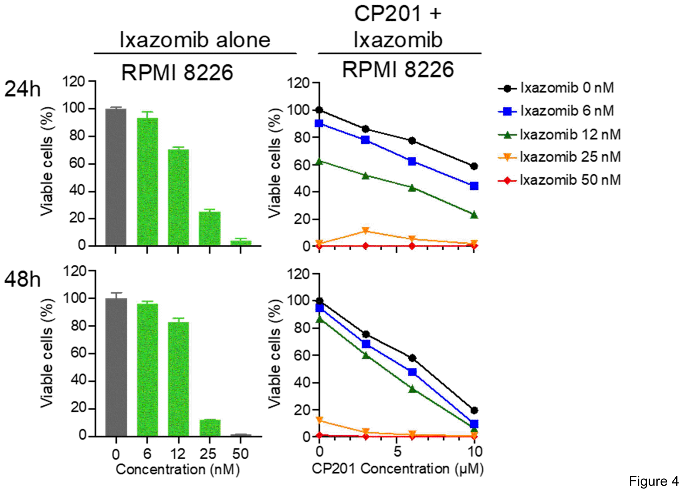

Figure 4 shows in vitro efficacy of ixazomib (0, 6, 12, 25 and 50 nM) alone and in combination with S-CP201 (0, 3, 6 and 10 µM) in 1 MM cell line (RPMI-8226) at 24 and 48 hours (CP201=S-CP201; Tested concentrations of ixazomib alone: 0, 6, 12, 25, and 50 nM; Tested concentrations of S-CP201 in combination with ixazomib: 0, 3, 6 and 10 µM).

In conclusion, a remarkable in vitro anti-myeloma activity of single-agent S-CP201 was seen. The combination of S-CP201 with the approved myeloma-targeting drugs dexamethasone, selinexor, and ixazomib led to a markedly enhanced and additive or, in the case of dexamethasone even a synergistic, effect of the two-agent combinations. Of note, these data indicated that S-CP201 is capable of reverting steroid-refractory myeloma cells to a sensitive state as long as the basic requirements for steroid-responsiveness, such as expression of the glucocorticoid receptor, are still fulfilled.

### Literature

Albagoush SA, Shumway C, Azevedo AM: Multiple myeloma. StatPearls [Internet], 2025; updated 30 January 2023; https://www.ncbi.nlm.nih.gov/books/NBK534764/.
Bausert WRE, Berdel WE, Munder PG: The effect of lysophospholipids on the growth of different murine tumors. Abstract. Verh. Dtsch. Krebsges. 3: 532, 1982.
Cowan AJ, Green DJ, Kwok M, Lee S, Coffey DG, Holmberg LA, Tuazon S, Gopal AK, Libby EN: Diagnosis and management of multiple myeloma. A review. JAMA 327 (5): 464-477, 2022.
Gajate C, Mollinedo F: Edelfosine and perifosine induce selective apoptosis in multiple myeloma by recruitment of death receptors and downstream signaling molecules into lipid rafts. Blood 109 (2): 711-1719, 2007.
Glasser L. Dalton WS, Fiederlein RL, Cook P, Powis G, Vogler WR: Response of human multiple myeloma-derived cell line to alkyl-lysophospholipid. Experimental Hematology 24: 253-257, 1996.
Holstein SA, Suman VJ, Hillengass J, McCarthy PL: Future directions in maintenance therapy in multiple myeloma. J Clin Med 10(11), 2261: 1-15, 2021.
Mollinedo F, de la Iglesia-Vicente J, Gajate C, Estella-Hermoso de Mendoza A, Villa-Pulgarin JA, Campanero MA, Blanco-Prieto MJ: Lipid raft-targeted therapy in multiple myeloma. Oncogene 29, 3748-3757, 2010.
Offidani M, Corvatta L, Morè S, Olivieri A: Novel experimental drugs for treatment of multiple myeloma. Journal of Experimental Pharmacology 13: 245-264, 2021.
Rajkumar SV: Multiple myeloma: 2024 update on diagnosis, risk-stratification, and management. Annual Clinical Updates in Hematological Malignancies 99 (9): 1802-1824, 2024.
Pinto V, Bergantim R, Caires HR, Seca H, Guimarães JE, Vasconcelos MH: Multiple myeloma: Available therapies and causes of drug resistance. Cancers (Basel) 12(2), 407: 1-32, 2020.
Rosenberg AS: From mechanism to resistance - changes in the use of dexamethasone in the treatment of multiple myeloma. Leuk Lymphoma 64(2): 283-291, 2023.
Uckun FM: Cancer drug resistance in multiple myeloma. Cancer Drug Resist 5: 271-276, 2022.

## Claims

1. Edelfosine for use in the treatment of multiple myeloma.

2. Edelfosine for use according to claim 1, wherein the edelfosine is (S)-edelfosine.

3. Edelfosine for use according to claim 1 or 2, wherein edelfosine is used in combination with corticosteroids (e.g., dexamethasone, prednisone), proteasome inhibitors (e.g., bortezomib, carfilzomib, ixazomib), cytotoxic drugs (e.g., cyclophosphamide, doxorubicin, bendamustine, melphalan), immunomodulators (e.g., lenalidomide, pomalidomide, thalidomide), stem cell mobilizing drugs (e.g., motixafortide, plerixafor), nuclear export inhibitors/exportin 1 (XPO1) inhibitors (e.g., selinexor), Bcl-2 inhibitors (e.g., venetoclax), bisphosphonates (e.g., zoledronic acid), SLAMF7 (CD319)-directing drugs (e.g., elotuzumab), histone deacetylase inhibitors (e.g., panobistat), C38-directing drugs (e.g., belantamab, daratumumab, isatuximab), BCMA-directed CAR-T cell treatments (e.g., ciltacabtagene autoleucel, idecabtagene vicleucel), BMCA/CD3 directed bispecific antibodies/T cell engagers (e.g., elranatamab, teclistamab, linvoseltamab), GPRC5D/CD3-directed bispecific antibodies/T cell engagers (e.g., talquetamab), PD-1/PD-L1 inhibitors (e.g., pembrolizumab), Pi3K inhibitors (e.g., pictilisib), BRAF inhibitors, RAS inhibitors, MEK inhibitors, and/or MYC-degrading and inhibiting drugs.

4. Edelfosine for use according to claim 1 or 2, wherein edelfosine is used in combination with dexamethasone, selinexor, and/or ixazomib.

5. Edelfosine for use according to claim 1 or 2, wherein edelfosine is used in combination with dexamethasone.

6. Edelfosine for use according to any one of the preceding claims, wherein the multiple myeloma is selected from frontline and advanced or recurrent stages of multiple myeloma.

7. Edelfosine for use according to any one of the preceding claims, wherein edelfosine is used in the treatment of multiple myeloma in standard-risk and high-risk multiple myeloma patients.

8. Edelfosine for use according to any one of the preceding claims, wherein edelfosine is used as induction treatment for autologous stem cell transplantation (ASCT).

9. Edelfosine for use according to any one of the preceding claims, wherein edelfosine is used as maintenance therapy for multiple myeloma.

10. Edelfosine for use according to any one of the preceding claims, wherein edelfosine is used in resensitizing dexamethasone-refractory multiple myeloma.
